# EUROPÄISCHE PATENTANMELDUNG

(11) **EP 3 263 577 A1**
(43) Veröffentlichungstag der Anmeldung: **03.01.2018**
(21) Anmeldenummer: 16178107.5
(22) Anmeldetag: 06.07.2016
(51) Int. Cl.: C07F 15/00, C07F 15/06

(54) **KOMPLEXE VON DIPHENYLSELENONEN, DEREN VERWENDUNG UND KATALYSEVERFAHREN**

(30) Priorität: 27.06.2016 DE 102016211499; 27.06.2016 DE 102016211501; 27.06.2016 DE 102016211500
(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); SELENT, Detlef, 18059 Rostock (DE); WWEILBEER, Claudia, T6C 4C8 Edmonton (CA); BÖRNER, Armin, 18059 Rostock (DE)

(57) **Zusammenfassung**

Neue Komplexe von Diphenylselenonen sowie deren Verwendung und Verfahren in denen die Komplexe eingesetzt werden.

## Beschreibung

Neue Komplexe von Diphenylselenonen, sowie deren Verwendung und Verfahren in denen die Komplexe zur Katalyse eingesetzt werden.

Die Eignung von spezifischen Diphenylseleniden oder Diphenylselenoxiden zur Herstellung von Komplexen und deren Verwendung zur Hydroformylierung von Olefinen wurde kürzlich nachgewiesen und in noch nicht veröffentlichten Patentanmeldungen beschrieben.

Eine Syntheseroute zur Herstellung von Diphenylselenoxiden ist nachfolgend im experimentellen Teil offenbart. Die Herstellung von Diphenylseleniden wird auch in der europäischen Patentanmeldung EP15168377.8 beschrieben. In diesem Verfahren können Diphenylselenide hergestellt werden, indem mindestens ein erstes Aren und optional ein zweites Aren in Gegenwart von Selendioxid im Sauren, insbesondere in Gegenwart einer Säure mit einem pKs-Wert im Bereich von 0 bis 5, bei einer definierten Temperatur umgesetzt wird. So kann beispielsweise ein Aren oder ein substituiertes Aren in Essigsäure mit Selendioxid umgesetzt werden. Ein bevorzugter Temperaturbereich liegt bei 50 bis 120 °C. Die Reaktionsdauer, d.h. die Umsetzung, erfolgt vorzugweise über Tage. Die Arene können mit Wasserstoff, Alkyl und/oder Halogen substituiert sein. Die erhaltenen Diphenylselenide können anschließend zu Diphenylselenoxiden oxidiert werden.

In der Hydroformylierung werden bislang in der Regel Mono- und Bisphosphite eingesetzt, die oftmals aus Biphenol-Bausteinen aufgebaut sind. Die Entwicklung neuer Liganden ist häufig durch die zur Verfügung stehenden Ligandenbausteine limitiert. So stellen Diphenylselenone eine hochinteressante Verbindungsklasse dar, die ein großes Potential in Hydroformylierungsreaktionen verspricht

Aufgabe der Erfindung war es, eine weitere gänzlich neue Substanzklasse an Ligandenbausteinen bzw. Liganden und Katalysatoren herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren bereitzustellen oder herzustellen. Daher bestand auch die Aufgabe neue Ligandenbausteine bereitzustellen, die als Zwischenprodukt zur Herstellung von Liganden oder als Zwischenprodukt zur Herstellung von Komplexen dienen. Eine zusätzliche Aufgabe bestand darin Liganden oder Ligandenbausteine für eine gezielte Steuerung der Katalyse bereitzustellen.

Gelöst werden die Aufgaben durch Diphenylselenone die am Selen zweifach Arylfunktionalisiert sind. Ferner werden die Aufgaben gelöst durch Komplexe enthaltend diese Diphenylselenone. Die erfindungsgemäßen Diphenylselenone sind ausgewählt aus unsubstituierten oder substituierten Diphenylselenonen der Struktur **I**, sowie vorzugsweise der Strukturen **Ia, Ib, Ic, Ic1, Id** und **Ie,** als auch aus Gemischen enthaltend mindestens zwei der vorgenannten Strukturen.

Gegenstand der Erfindung ist ein Komplex umfassend
- mindestens ein Diphenylselenon der allgemeinen Struktur **I** 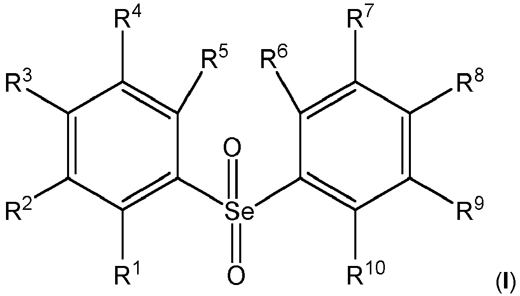 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl,
   -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- mindestens ein Metallatom ausgewählt aus Rh, Ru, Co, Ir.

Erfindungsgemäß kann es bevorzugt sein, wenn alle Alkylgruppen unsubstituiert sind. Besonders bevorzugte Komplexe umfassen als mindestens ein Metallatom Rh, Ir, Ru, wobei Rh besonders bevorzugt ist.

In einer Ausführungsform umfasst der Komplex ein Diphenylselnon der allgemeinen Struktur **Ia:** 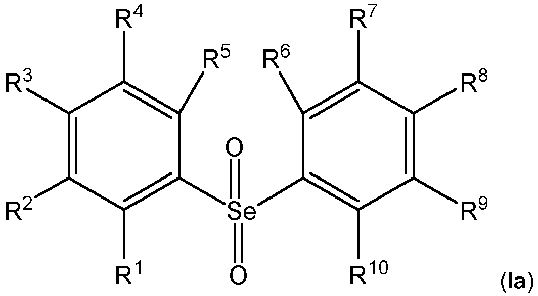
(i) worin R¹, R³, R⁵, R⁶, R⁸ und R¹⁰ jeweils -H und R², R⁴, R⁷ und R⁹ jeweils unabhängig ausgewählt sein können aus -(C₁-C₁₂)-Alkyl, mit Alkyl linear, verzweigt oder cyclisch, oder
(ii) worin R¹, R³, R⁵, R⁶, R⁸ und R¹⁰ jeweils ausgewählt sein können aus -(C₁-C₁₂)-Alkyl, mit Alkyl linear, verzweigt oder cyclisch, und R², R⁴, R⁷ und R⁹ jeweils -H, oder
(iii) worin R³ und R⁸ jeweils ausgewählt sein können aus -O-(C₁-C₁₂)-Alkyl, -O-(C₆-C₂₀)-Aryl, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, und -Halogen, insbesondere -O-(C₂-C₁₂)-Alkyl, bevorzugt -O-(C₃-C₁₂)-Alkyl, mit Alkyl linear, verzweigt oder cyclisch, und R¹, R², R⁴, R⁵, R⁶, R⁷, R⁹ und R¹⁰ jeweils -H, oder
(iv) worin R¹, R², R³, R⁴, R⁵, R⁶, R⁷, R⁸, R⁹, R¹⁰ jeweils unabhängig voneinander ausgewählt sein können aus -Halogen, insbesondere ist Halogen Fluor oder Chlor,
wobei in (i), (ii) (iii) oder (iv) die Alkyl- und Arylgruppen jeweils unabhängig unsubstituiert oder substituiert sein können, substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

In einer Ausführungsform umfasst der Komplex ein Diphenylselnon der allgemeinen Struktur **Ib:**

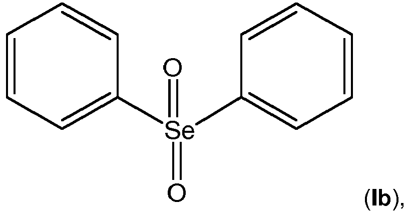

In einer Ausführungsform umfasst der Komplex ein Diphenylselnon der allgemeinen Struktur **Ic:** 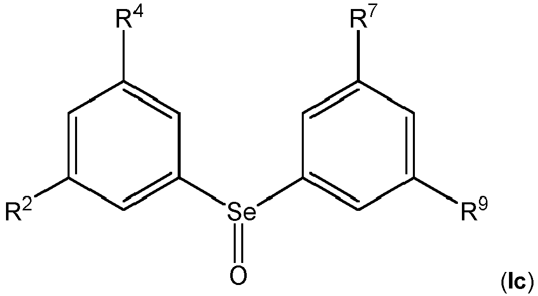 wobei in der Struktur **Ic** R², R⁴, R⁷ und R⁹ jeweils unabhängig ausgewählt sind aus -(C₁-C₁₂)-Alkyl.

In einer Ausführungsform umfasst der Komplex ein Diphenylselnon der allgemeinen Struktur **Ic1:**

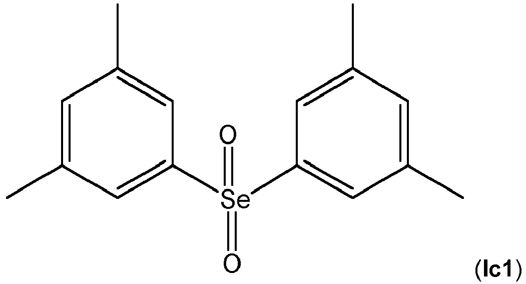

In einer Ausführungsform umfasst der Komplex ein Diphenylselnon der allgemeinen Struktur **Id:**

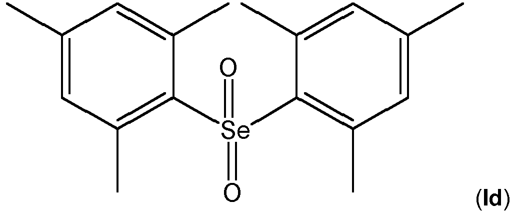

In einer Ausführungsform umfasst der Komplex ein Diphenylselnon der allgemeinen Struktur **Ie:** 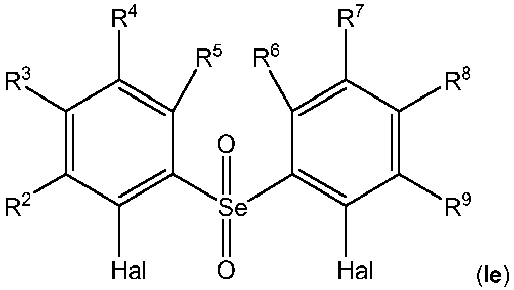 mit R², R³, R⁴, R⁵, R⁶, R⁷, R⁸ und R⁹ jeweils unabhängig ausgewählt aus -H, -(C₁-C₁₂)-Alkyl und -Halogen.

Die Begriffe Diphenylselenon, Diphenylselenid, Diphenylselenoxid und Olefin werden in dieser Anmeldung als Gattungsbegriff verwendet und umfassen jeweils somit neben den unsubstituierten auch die substituierten Verbindungen.

Dabei umfassen besonders bevorzugte Komplexe nach einer Alternative mindestens ein Metallatom ausgewählt aus Rh, Ru, Co, Ir, insbesondere Rh, Ir, Ru, bevorzugt Rh, und mindestens ein Diphenylselenoxid der allgemeinen Struktur **I** 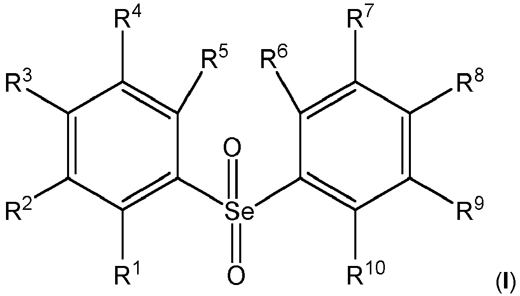
a) mit R¹, R³, R⁵, R⁶, R⁸ und R¹⁰ jeweils -H und R², R⁴, R⁷ und R⁹ jeweils unabhängig ausgewählt aus -(C₁-C₁₂)-Alkyl, wobei die Alkylgruppen linear, verzweigt oder cyclisch sein können, optional sind die Alkylgruppen mit mindestens einer -(C₃-C₁₂)-Cycloalkylgruppe substituiert, oder
b) mit R¹, R³, R⁵, R⁶, R⁸ und R¹⁰ jeweils unabhängig ausgewählt aus -(C₁-C₁₂)-Alkyl, wobei die Alkylgruppen linear, verzweigt oder cyclisch sein können, optional sind die Alkylgruppen mit mindestens einer -(C₃-C₁₂)-Cycloalkylgruppe substituiert,
   und R², R⁴, R⁷ und R⁹ jeweils -H.

Nach weiteren besonderen Alternativen sind Gegenstand der Erfindung Komplexe umfassend mindestens ein Metallatom ausgewählt aus Rh, Ru, Co, Ir, insbesondere Rh, Ir, Ru, bevorzugt Rh, und in denen das mindestens eine Diphenylselenon der allgemeinen Struktur **I** unabhängig ausgewählt ist aus einer der Struktur la, **Ib, Ic, Ic1** oder **Id.**

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt um -(C₁-C₆)-Alkylgruppen. Beispiele für -(C₁-C₁₂)-Alkylgruppen sind, insbesondere Methyl, Ethyl, Propyl, Isopropyl, n-Butyl, iso-Butyl, sec.-Butyl, *tert*.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Diemthylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl-, Decyl. Halogen (Hal) umfasst Chlor, Brom, Fluor und Jod, wobei Chlor und Fluor besonders bevorzugt sind.

Die Erläuterungen zum Ausdruck -(C₁-C₁₂)-Alkyl gelten für alle Alkylgruppen.

Gemäß einer Ausführungsvariante können R¹, R², R³, R⁴, R⁵, R⁶, R¹, R⁸, R⁹ und R¹⁰, die jeweils unabhängig einem substituierten Alkyl mit mindestens einer -(C₃-C₁₂)-Cycloalkylgruppe entsprechen, als -(C₃-C₁₂)-Cycloalkyl im Sinne der vorliegenden Erfindung umfassen, mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl. Ein Beispiel für ein substituiertes Cycloalkyl wäre Menthyl.

Besonders bevorzugte Komplexe umfassen mindestens ein Rhodium-Atom sowie mindestens ein Diphenylselenon der Struktur **I, Ia, Ib, Ic, Ic1** und/oder **Id,** wobei vorzugsweise in der Struktur **I** R¹, R⁵, R⁶ und/oder R¹⁰ jeweils unabhängig ausgewählt sind aus -H oder -(C₁-C₁₂)-Alkyl, mit Alkyl linear, verzweigt oder cyclisch, bevorzugt sind in der Struktur **Ia** R¹, R⁵, R⁶ und/oder R¹⁰ jeweils gleich und ausgewählt aus -H oder -(C₁-C₆)-Alkyl, mit Alkyl verzweigt oder cyclisch.

Besonders bevorzugte Komplexe umfassen mindestens ein Ruthenium-Atom sowie mindestens ein Diphenylselenon der Struktur **I, Ia, Ib, Ic, Ic1** und/oder **Id,** wobei vorzugsweise in der Struktur **I** R¹, R⁵, R⁶ und/oder R¹⁰ jeweils unabhängig ausgewählt sind aus -H oder -(C₁-C₁₂)-Alkyl, mit Alkyl linear, verzweigt oder cyclisch, bevorzugt sind in der Struktur **Ia** R¹, R⁵, R⁶ und/oder R¹⁰ jeweils gleich und ausgewählt aus -H oder -(C₁-C₆)-Alkyl, mit Alkyl verzweigt oder cyclisch.

Besonders bevorzugte Komplexe umfassen mindestens ein Kobalt-Atom sowie mindestens ein Diphenylselenon der Struktur **I**, **Ia**, **Ib, Ic, Ic1** und/oder **Id,** wobei vorzugsweise in der Struktur **I** R¹, R⁵, R⁶ und/oder R¹⁰ jeweils unabhängig ausgewählt sind aus -H oder -(C₁-C₁₂)-Alkyl, mit Alkyl linear, verzweigt oder cyclisch, bevorzugt sind in der Struktur **Ia** R¹, R⁵, R⁶ und/oder R¹⁰ jeweils gleich und ausgewählt aus -H oder -(C₁-C₆)-Alkyl, mit Alkyl verzweigt oder cyclisch.

Besonders bevorzugte Komplexe umfassen mindestens ein Iridium-Atom sowie mindestens ein Diphenylselenon der Struktur **I, Ia, Ib, Ic, Ic1** und/oder **Id,** wobei vorzugsweise in der Struktur **I** R¹, R⁵, R⁶ und/oder R¹⁰ jeweils unabhängig ausgewählt sind aus -H oder -(C₁-C₁₂)-Alkyl, mit Alkyl linear, verzweigt oder cyclisch, bevorzugt sind in der Struktur **Ia** R¹, R⁵, R⁶ und/oder R¹⁰ jeweils gleich und ausgewählt aus -H oder -(C₁-C₆)-Alkyl, mit Alkyl verzweigt oder cyclisch.

Besonders bevorzugt sind R², R⁴, R⁷, R⁹ in der Struktur **I** jeweils unabhängig voneinander ausgewählt aus -H, -(C₁-C₁₂)-Alkyl und -Halogen und R¹, R³, R⁵, R⁶, R⁸, R¹⁰ sind -H. Besonders bevorzugt sind R², R⁴, R⁷, R⁹ in der Struktur **I** jeweils unabhängig voneinander ausgewählt aus -H und R¹, R³, R⁵, R⁶, R⁸, R¹⁰ sind jeweils unabhängig ausgewählt aus -H, -(C₁-C₁₂)-Alkyl und -Halogen.

Besonders bevorzugt sind Diphenylselenone der Struktur **I** mit R¹, R⁵, R⁶ und R¹⁰, die vorzugsweise jeweils gleich sind und ausgewählt sind aus -H oder -(C₁-C₁₂)-Alkyl, mit Alkyl linear, verzweigt oder cyclisch, vorzugsweise ist Alkyl ausgewählt aus Methyl, Ethyl, Isopropyl, *tert*.-Butyl, iso-Pentyl, 1,1-Dimethylpropyl. Vorzugsweise sind R², R³, R⁴, R⁷, R⁸, R⁹ gleich -H.

Weitere bevorzugte Diphenylselenone weisen als R², R⁴, R⁷ und R⁹ Methyl oder tert. Butyl mit R³ und R⁸ beide gleich -Halogen, -H oder -(C₁-C₆)-Alkyl, insbesondere Methyl, Ethyl, Isopropyl, *tert*.-Butyl, iso-Pentyl, 1,1-Dimethylpropyl. Vorzugsweise sind R¹, R⁵, R⁶, R¹⁰ gleich -H.

Weitere bevorzugte Diphenylselenone weisen als R², R⁴, R⁷ und R⁹ Methyl mit R³ und R⁸ beide gleich -Halogen, -H oder -(C₁-C₆)-Alkyl, insbesondere Methyl, Ethyl, Isopropyl, *tert.-*Butyl, iso-Pentyl, 1,1-Dimethylpropyl. Vorzugsweise sind R¹, R⁵, R⁶, R¹⁰ gleich -H.

Besonders bevorzugt sind R¹, R², R³, R⁴, R⁵, R⁶, R⁷, R⁸, R⁹ und R¹⁰ in Diphenylselenonen der Struktur **I** jeweils unabhängig voneinander ausgewählt aus -H und -(C₁-C₁₂)-Alkyl, insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl.

Besonders bevorzugt sind R¹, R², R³, R⁴, R⁵, R⁶, R⁷, R⁸, R⁹ und R¹⁰ in Diphenylselenonen der Struktur **I** jeweils unabhängig voneinander ausgewählt aus -(C₁-C₁₂)-Alkyl, insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl.

Besonders bevorzugt sind R¹, R², R³, R⁴, R⁵, R⁶, R⁷, R⁸, R⁹, und R¹⁰ in Diphenylselenonen der Struktur **I** jeweils unabhängig voneinander ausgewählt aus -H und -Halogen, ausgewählt aus Chlor, Brom, Fluor, Iod.

Besonders bevorzugt sind R¹, R², R³, R⁴, R⁵, R⁶, R⁷, R⁸, R⁹ und R¹⁰ in Diphenylselenonen der Struktur **I** gleich -Halogen, ausgewählt aus Chlor, Brom, Fluor und Jod.

Das Diphenylselenon der Struktur **I** kann vorliegen mit R², R³, R⁴, R⁷, R⁸ und R⁹ die jeweils unabhängig ausgewählt sein können aus -H, -(C₁-C₁₂)-Alkyl, -Halogen, mit Alkyl linear, verzweigt oder cyclisch, wobei die Alkylgruppen jeweils unabhängig unsubstituiert sind oder optional kann ein vorgenanntes Alkyl mit mindestens einer -(C₃-C₁₂)-Cycloalkylgruppe substituiert sein,
wobei R¹, R⁵, R⁶ und R¹⁰ jeweils unabhängig ausgewählt sind aus -(C₁-C₁₂)-Alkyl und/oder -Halogen, mit Alkyl linear, verzweigt oder cyclisch und mindestens ein Metallatom ausgewählt aus Rh, Ru, Co, Ir. Erfindungsgemäß kann es bevorzugt sein, wenn alle Alkylgruppen unsubstituiert sind.

Das Diphenylselenon der Struktur **I** kann vorliegen mit R², R³, R⁴, R⁷, R⁸ und R⁹ die jeweils unabhängig ausgewählt sein können aus -H, -(C₁-C₁₂)-Alkyl, -Halogen, mit Alkyl linear, verzweigt oder cyclisch, wobei die Alkylgruppen jeweils unabhängig unsubstituiert sind oder optional kann ein vorgenanntes Alkyl mit mindestens einer -(C₃-C₁₂)-Cycloalkylgruppe substituiert sein, wobei R¹, R⁵, R⁶ und R¹⁰ jeweils unabhängig ausgewählt sind aus -H und/oder -Halogen, und mindestens ein Metallatom ausgewählt aus Rh, Ru, Co, Ir. Erfindungsgemäß kann es bevorzugt sein, wenn alle Alkylgruppen unsubstituiert sind.

Ferner ist Gegenstand der Erfindung ein Zwischenprodukt zur Herstellung von mindestens ein Metallatom enthaltenden Komplexen sowie dessen Verwendung zur Herstellung von mindestens ein Metallatom enthaltenden Komplexen, wobei das Zwischenprodukt mindestens ein Diphenylselenon der allgemeinen Struktur **I** umfassen kann 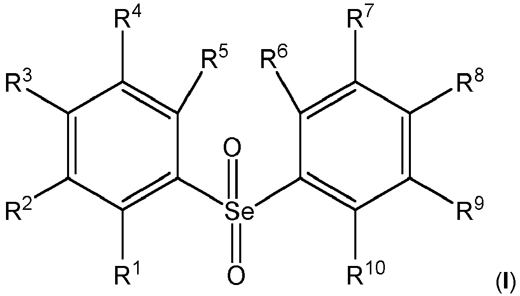 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können: substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl. Das Zwischenprodukt bzw. das Diphenylselenon kann identische Substitutionsmuster wie die Struktur **I, Ia, Ib, Ic, Ic1** oder **Id** im Komplex aufweisen. Gleichfalls Gegenstand der Erfindung ist ein Diphenylselenon der Struktur **I** wie vorstehen definiert.

Im Zwischenprodukt oder im Diphenylselenon der Struktur **I** sind R³ und R⁸ bevorzugt -O-(C₂-C₁₂)-Alkyl, bevorzugt -O-(C₃-C₁₂)-Alkyl. Ferner sind im Zwischenprodukt R³ und R⁸ bevorzugt -(C₂-C₁₂)-Alkyl, bevorzugt -(C₃-C₁₂)-Alkyl. Ferner sind im Zwischenprodukt oder im Diphenylselenon der Struktur **I** R³ und R⁸ bevorzugt -Fluor oder Brom. Wobei in diesen Alternativen R¹, R², R⁴, R⁵, R⁶, R⁷ , R⁹, R¹⁰ jeweils unabhängig wie vorstehend definiert sind. Zwischenprodukt oder im Diphenylselenon der Struktur I sind vorzugsweise nicht alle Reste gleichzeitig gleich H, d.h. R¹, R², R⁴, R⁵, R⁶, R⁷, R⁹ und R¹⁰ sind vorzugsweise nicht gleichzeitig gleich -H.

Im einem bevorzugten Zwischenprodukt oder im Diphenylselenon der Struktur **I** sind R¹, R³, R⁵, R⁶, R⁸, R¹⁰ bevorzugt -(C₂-C₁₂)-Alkyl, bevorzugt -(C₃-C₁₂)-Alkyl und R², R⁴, R⁷, R⁹, vorzugsweise -H.

Im Zwischenprodukt oder im Diphenylselenon der Struktur **I** sind R¹, R², R³, R⁴, R⁵, R⁶, R⁷, R⁸, R⁹, R¹⁰ jeweils unabhängig ausgewählt aus Halogen, insbesondere aus Fluor und Chlor, bevorzugt sind R¹, R², R³, R⁴, R⁵, R⁶, R⁷, R⁸, R⁹, R¹⁰ gleich Fluor oder gleich Chlor.

Gleichfalls Gegengenstand der Erfindung ist ein Verfahren zur Herstellung des Diphenylselenon der Struktur **I**, wie oben definiert, indem ein Diphenylselenid der allgemeinen Struktur **III,** 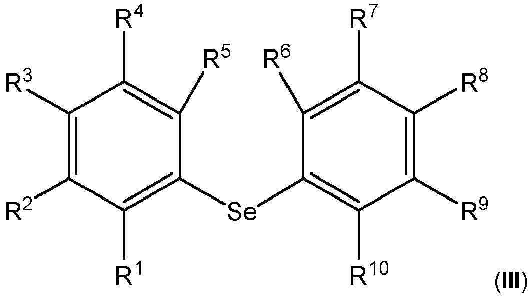 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- in einem ersten aprotischen, polaren, organischen Lösemittel, in Gegenwart eines Alkalisalzes des Hypochlorids oder einem Alkalisalz des Hypobromids zu einem Reaktionsgemisch umgesetzt wird, und
- das Reaktionsgemisch mit einem zweiten organischen Lösemittel unter Ausbildung eines zweiphasigen Gemisches behandelt wird, wobei sich das Diphenylselenon der Struktur **I**, in dem zweiten organischen Lösemittel löst,
- Abtrennen des zweiten organischen Lösemittels enthaltend Diphenylselenon der Struktur **I,**
- Entfernen des zweiten organischen Lösemittels und
- Erhalten des Diphenylselenons der Struktur **I**.

Gleichfalls Gegengenstand der Erfindung ist ein Verfahren zur Herstellung des Diphenylselenon der Struktur **I,** wie oben definiert, indem ein Diphenylselenoxid der allgemeinen Struktur **II**, 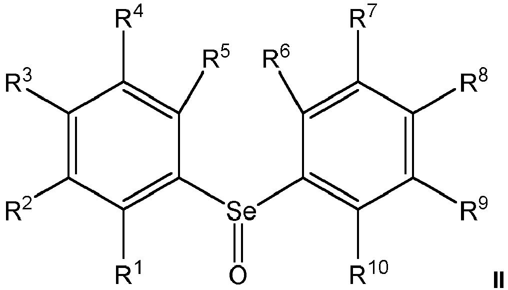 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- in einem aprotischen, organischen Lösemittel in Gegenwart eines mit Alkyl-Gruppen funktionalisierten quartären- Ammoniumhalogenids mit einem Alkalisalz des Hypochlorids oder einem Alkalisalz des Hypobromids, zu einem Reaktionsgemisch umgesetzt wird, und
- das Reaktionsgemisch mit Wasser unter Ausbildung eines zweiphasigen Gemisches behandelt wird, wobei das Diphenylselenon der Struktur **I**, im aprotischen organischen Lösemittel verbleibt,
- Abtrennen des aprotischen, organischen Lösemittels enthaltend Diphenylselenon der Struktur **I,**
- Entfernen des aprotischen, organischen Lösemittels und
- Erhalten des Diphenylselenons der Struktur **I**.

Als quartäres- Ammoniumhalogenid wird vorzugsweise (R¹¹)₄N⁺Hal⁻ verwendet, wobei R¹¹ gleich -(C₁-C₁₂)-Alkyl sein kann. Besonders bevorzugt ist R¹¹ gleich n-(C₁-C₁₂)-Alkyl und Hal⁻ gleich Chlorid (Cl⁻) oder Bromid (Br⁻).

Gleichfalls Gegenstand der Erfindung ist die Verwendung eines Diphenylselenons der allgemeinen Struktur **I** 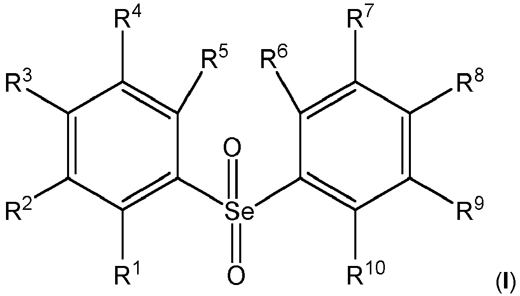 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
a) als Ligand in einem Komplex umfassend mindestens ein Metallatom oder
b) als Zwischenprodukt zur Herstellung von Liganden oder als Zwischenprodukt zur Herstellung von Komplexen.

Die Verwendung zur Herstellung von Zwischenprodukten kann für Strukturen **I** mit mindestens einem Rest ausgewählt aus R¹, R⁵, R⁶ und R¹⁰ gleich Halogen bevorzugt sein. Ferner ist Gegenstand der Erfindung die Verwendung eines Komplexes umfassend mindestens ein Diphenylselenon der allgemeinen Struktur **I** in einer Hydroformulierungreaktion.

Ebenfalls Gegenstand der Erfindung ist ein Verfahren umfassend die Verfahrensschritte
(i) Vorlegen mindestens eines Olefins,
(ii) Zugabe eines Komplexes umfassend
   - mindestens ein Diphenylselenon, welches eine allgemeine Struktur **I** aufweist 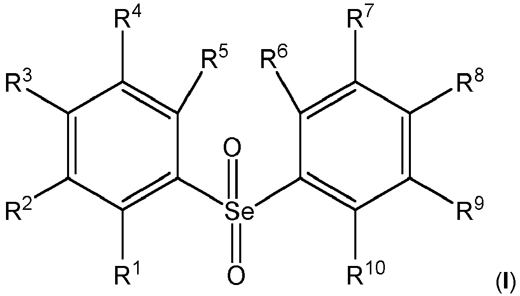 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
   - mindestens ein Metallatom, das ausgewählt sein kann aus Rh, Ru, Co, Ir, und/oder
   - Zugabe mindestens eines Diphenylselenons der allgemeinen Struktur **I,** und einer Substanz, welche ein Metallatom ausgewählt aus Rh, Ru, Co, Ir umfassen kann,
(iii) Zuführen von H₂ und CO,
(iv) Erwärmen des Reaktionsgemisches,
wobei das Olefin zu einem Aldehyd umgesetzt wird.

Die Verfahrensschritte (i), (ii), (iii) und (iv) können alternativ in einer beliebigen Reihenfolge durchgeführt werden. Bevorzugt kann in dem Verfahren ein Komplex eingesetzt werden umfassend mindestens ein Diphenylselenon der Struktur **I**, **Ia**, **Ib, Ic, Ic1** oder **Id** oder es können mindestens ein entsprechendes Diphenylselenon der Struktur **I**, **Ia**, **Ib, Ic, Ic1** oder **Id** und eine Substanz, welche ein vorgenanntes Metallatom aufweist, eingesetzt werden.

Dabei kann vorzugsweise mit den erfindungsgemäßen Verbindungen der Strukturen **I, Ia, Ib, Ic, Ic1** oder **Id** in einer Hydroformylierung gemäß vorstehender Verwendung oder vorstehendem Verfahren eine Ausbeute von größer gleich 80 %, insbesondere 85 % und/oder eine n-Regioselektivität von größer 20 %, insbesondere größer gleich 25 % erzielt werden, wobei vorzugsweise in Struktur **I** R¹ bis R¹⁰ jeweils unabhängig ausgewählt sind aus -H, -(C₁-C₁₂)-Alkyl und Halogen, mit Alkyl linear, verzweigt oder cyclisch sind.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter,* Art.-Nr. BIE 073107033), Ethylacetat (99.5%, Fa. *Walter,* Art.-Nr. BIE 003917025) und n-Hexan (95%, Fa. *Walter (Baker),* Art.-Nr. 8669), n-Heptan (95%, Fa. *Walter (Baker),* Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-d₈ (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co.* KG verwendet. Diese wurden, sofern nicht anders vermerkt, ohne weitere Reinigung eingesetzt.

### Chromatographische Methoden

Die Säulenchromatographie: Säulenchromatographische Trennungen wurden bei erhöhtem Druck (Flash-Chromatographie) an Silicagel 60 230-400 mesh der Firma *Merck KGaA* (Korngröße: 0.040-0.063 mm) durchgeführt. Die verwendeten Eluentengemische sowie die Volumenverhältnisse v/v sind in den nachfolgenden Vorschriften angegeben. Für die verwendeten Eluenten gelten folgende Abkürzungen: DCM (Dichlormethan), EE (Ethylacetat), H (n-Hexan), sowie Tol (Toluol).

Filtration: Filtrationen zur Abtrennung von entstandenen Feststoffen wurden mit einer G4-Fritte (Porenweite: 10-16 µm) durchgeführt.

### Analytik

¹H-NMR-Spektroskopie: Die ¹H-NMR-Spektren wurden mit dem Modell *AV 300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈: δ = 7.09; 7.00; 6.98; 2.09 ppm) dienten dabei als Standard.
¹³C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈: δ = 137.9; 129.2; 128.3; 125.5; 20.4 ppm), wobei die chemischen Verschiebungen den ¹H-breitbandentkoppelten Spektren entnommen wurden.
⁷⁷Se-NMR-Spektroskopie: Die ⁷⁷Se-NMR-Spektren wurden mit dem *AV 300* (57 MHz) der Firma *Bruker* aufgenommen. Die Spektren wurden ¹H-breitbandentkoppelt vermessen. Die chemischen Verschiebungen sind in ppm angegeben.
Massen spektrometrie: EI-Massespektren wurden am Gerät *Finnigan MAT95-XP* der Firma *Thermo Electron* sowie ESI-TOF-Massespektren mit dem Modell *6210 Time-of-Flight LC*/*MS* der Firma *Agilent* aufgenommen.

### Autoklaven-Versuche der Rhodium-katalysierten Hydroformylierung

Die Hydroformylierung wurde in einem mit Druckkonstanthaltung, Gasflussmessung, Begasungsrührer und Druckpipette ausgestattetem 200 mL-Autoklaven der Fa. *Premex Reactor AG,* Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat n-Octen (*EVONIK Industries AG,* Octenisomerengemisch aus 1-Octen: 3,3 %; *cis*+*trans-*2-Octen: 48.5%; *cis+trans-*3-Octen: 29.2%; *cis*+*trans*-Octen-4: 16.4%; gerüstisomere Octene: 2.6%) wurde mehrere Stunden über Natrium am Rückfluss erhitzt und unter Argon destilliert.
Für die Versuche wurden im Autoklaven unter Argonatmosphäre Lösungen der Katalysatorvorstufe und des Liganden gemischt. Als Katalysatorvorstufe kam [(acac)Rh(COD)] (*Umicore,* acac=Acetylacetonat-Anion; COD=1,5-Cyclooctadien) zum Einsatz. Für Versuche mit einer Konzentration von 100 ppm-m Rhodium wurden 10 mL einer 4.31 mM Lösung in den Autoklaven gegeben. Anschließend wurde die einem Verhältnis L/Rh = 5:1 (bzw. 1:1) entsprechende Masse des Liganden in 10 mL Toluol gelöst und zugemischt. Durch Zugabe von weiterem Toluol wurde das Anfangsvolumen der Katalysatorlösung auf 41.0 ml eingestellt. In eine druckfeste Pipette wurde eingefüllt: n-Octen (10.70 g). Der Autoklav wurde bei einem Gesamtgasdruck (Synthesegas: *Linde;* H₂ (99.999%): CO (99.997%) = 1:1) von 42 bar für einen Enddruck von 50 bar unter Rühren (1500 U/min) auf die jeweils angegebenen Temperaturen aufgeheizt. Nach Erreichen der Reaktionstemperatur wurde der Synthesegasdruck auf 48.5 bar für einen Enddruck von 50 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. *Bronkhorst,* NL) über 4h geführt. Der Autoklav wurde nach Ablauf der Reaktionszeit auf Zimmertemperatur abgekühlt, unter Rühren entspannt und mit Argon gespült. Jeweils 1.0 mL der Reaktionsmischungen wurden unmittelbar nach Abschalten des Rührers entnommen, mit 5.0 mL Pentan verdünnt und gaschromatographisch analysiert: HP 5890 Series II plus, PONA, 50 m x 0.2 mm x 0.5 µm.
**Abkürzungen:** ber. = berechnet; gef. = gefunden; RT = Raumtemperatur

Die Verbindung **IIa** (CAS 7304-91-8) ist literaturbekannt. **IIa** kann hergestellt werden nach: Canadian Journal of Chemistry, 88, 906-909, 2010 oder ist erhältlich bei Alfa Aesar, 98%, CAS = 1132-39-4.

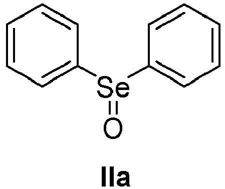

Diphenylselenide der Struktur **III** können entsprechend der europäischen Patentanmeldung EP15168377.8 hergestellt werden. Nach diesem Verfahren können Diphenylselenide hergestellt werden, indem mindestens ein erstes Aren und optional eines zweiten Arens in Gegenwart von Selendioxid im Sauren, insbesondere in Gegenwart einer Säure mit einem pKs-Wert im Bereich von 0 bis 5, beispielsweise Essigsäure, bei einer definierten Temperatur umgesetzt wird. Ein bevorzugter Temperaturbereich liegt bei 50 bis 120 °C. Die Reaktionsdauer, d.h. die Umsetzung erfolgt vorzugweise über Tage. Die Arene können mit Wasserstoff, Alkyl und/oder Halogen substituiert sein.

### Synthese von Diphenylselenoxiden II, insbesondere IIa.

Analog der folgenden Synthesevorschrift können die Diphenylselenoxide der Struktur **II** gemäß den angegebenen Substituten hergestellt werden.
175 µL (234 mg, 1.00 mmol, 1.0 eq) Diphenylselenid **III** wurde mit 140 mg (1.05 mmol, 1.05 eq) *N*-Chlorsuccinimid umgesetzt. Nach extraktiver Aufarbeitung wurden 235 mg (0.940 mmol, 94%) der Titelverbindung **I** als farbloser Feststoff erhalten.
IR (ATR): (cm-1) = 3044; 3008; 2989; 2941; 1570; 1470; 1437; 1300; 1156; 1069; 1056; 1047; 1017; 993; 915; 850; 820; 733; 686; 611; 481; 442, 1H-NMR (300 MHz, Toluol-d8): δ (ppm) = 7.67-7.51 (m, 4H, Ar-CH); 7.16-6.87 (m, 6H, Ar-CH); 13C-NMR (75 MHz, Toluol-d8): δ (ppm) = 145.1; 130.5; 129.3; 126.0; 77Se-NMR (57 MHz, Toluol-d8): δ (ppm) = 851.0; HR-MS (ESI-TOF): ber. für C12H11OSe ([M+H]+): 250.99700, gef.: 250.99691;
ber. für C12H10OSeNa ([M+Na]+): 272.97894, gef.: 272.97888; C12H10OSe (249.99 g/mol). Die analytischen Daten stimmen mit den Literaturdaten überein.

### Synthese von oxidierten Organoselenverbindungen

Die Synthesen von Diphenylselenoxid **IIa** und -dioxid **Ia** erfolgte, indem ein Selenid mit NCS (N-Chlorsuccinimid) oder NBS (N-Bromsuccinimid) oxidiert wurde. Die NaOCl-Oxidation in DMF konnte aufgrund der erfindungsgemäßen Modifikation des Verfahrens mit deutlich verbesserten Ausbeuten durchgeführt werden. ^{[}Ein weiteres erfindungsgemäßes Herstellverfahren umfasst die Oxidation mit NaOCl in einem Zweiphasensystem. Die Produkte wurden mittels z.B. ⁷⁷Se-NMR charakterisiert.

### Synthese von Diphenylselenon Ia

### a) ausgehend von Diphenylselenid IIIa

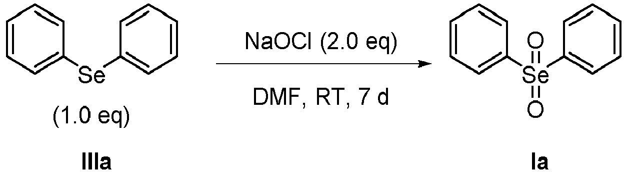

In einem 50 mL Zweihalskolben wurden 174 µL (234 mg, 1.00 mmol 1.0 eq) Diphenylselenid **IIIa** in 5.0 mL DMF vorgelegt. Anschließend wurden bei RT zu der gelben Lösung 1.49 mL (2.00 mmol, 2.0 eq, 10%ig in H₂O) NaOCl addiert, wobei eine Orange-Färbung mit gleichzeitiger Trübung der Lösung beobachtet wurde. Nach einer Reaktionszeit von 7 d wurden 10 mL Ethylacetat addiert und die entstandenen Phasen getrennt. Die organische Phase wurde zweimal mit je 20 mL H₂O gewaschen, die wässrige Phase zweimal mit je 15 mL Ethylacetat extrahiert und die vereinigten organischen Phasen über Magnesiumsulfat getrocknet. Das Trockenmittel wurde abfiltriert und das Lösungsmittel unter vermindertem Druck entfernt. Umkristallisation in *n*-Hexan/CH₂Cl₂ (4:1) und Trocknen des Produktes für 3 h im Vakuum bei 50°C ergab 228.4 mg (0.859 mmol, 86%) der Titelverbindung **Ia** als farblosen Feststoff.

### b) ausgehend von Diphenylselenoxid Ia

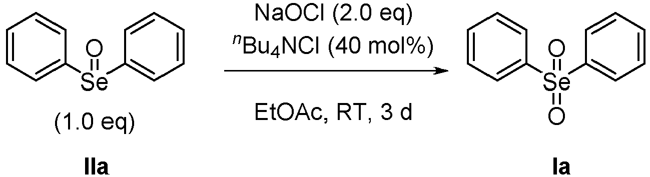

In einem 50 mL Zweihalskolben wurden 59.0 mg (0.212 mmol, 0.416 eq) tetra-n-Butylammoniumchlorid in 2.0 mL Ethylacetat vorgelegt. Zu der farblosen Lösung wurden 127.5 mg (0.510 mmol, 1.0 eq) Seleninyldibenzen **IIa** bei RT zugetropft, wobei eine Verfärbung zu einer schwach lavendel-farbigen Lösung beobachtet wurde. Anschließend wurden 755 µL (1.02 mmol, 2.0 eq, 10%ig in H₂O) NaOCl addiert, welches in einer hellgelben Verfärbung der Lösung resultierte. Nach einer Reaktionszeit von 3 d wurden 5.0 mL H₂O addiert und die entstandenen Phasen getrennt. Die organische Phase wurde zweimal mit je 20 mL H₂O gewaschen und die wässrige Phase zweimal mit je 15 mL Ethylacetat extrahiert. Die vereinigten organischen Phasen wurden über Magnesiumsulfat getrocknet. Das Trockenmittel wurde abfiltriert und das Lösungsmittel unter vermindertem Druck entfernt. Umkristallisation in *n*-Hexan/CH₂Cl₂ (4:1) und Trocknen des Produktes für 3 h im Vakuum bei 50°C ergab 125 mg (0.469 mmol, 92%) der Titelverbindung **Ia** als farblosen Feststoff. 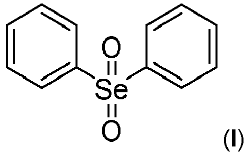 ¹H-NMR (300 MHz, CD₂Cl₂): δ (ppm) = 7.92-7.83 (m, 4H); 7.64-7.43 (m, 6H); ¹³C{¹H}-NMR (75 MHz, CD₂Cl₂): δ (ppm) = 142.8; 134.1; 130.3; 126.8; ⁷⁷Se{¹H}-NMR (57 MHz, CD₂Cl₂): δ (ppm) = 963.0; HR-MS (ESI-TOF): m/z: ber. für C₁₂H₁₁O₂Se ([*M*+H]⁺): 266.99192, gef.: 266.99188; ber. für C₁₂H₁₀O₂SeNa ([*M*+*Na*]⁺): 288.92386, gef.: 288.97414; C₁₂H₁₀O₂Se (265.99).

Die Synthese von Diphenylselendioxid **Ia** kann ebenso durch folgende Methoden realisiert werden:
a) mit HOF·MeCN (für Selen-Verbindungen): S. Potash, S. Rozen, Eur. J. Org. Chem. 2013, 5574-5579.
b) durch Photooxygenierung (für Selen- und Schwefelverbindungen): N. Sofikiti, C. Rabalakos, M. Stratakis, Tetrahedron Lett. 2004, 45, 1335-1337.
c) mit FeCl₃/H₂O₂ in Analogie zu den Schwefel-Verbindungen: G.A. Kraus, B.S. Fulton, J. Org. Chem. 1985, 50, 1784-1786.
d) mit ZnCl₂/H₂O₂ in Analogie zu den Schwefel-Verbindungen: J.-B. Feng, J.-L. Gong, X.-F. Wu, RSC Adv. 2014, 4, 29273-29275.

Eine bekannte Methode zur Oxidation von Diphenylselenid **III,** insbesondere **IIIa** zur Oxidationsstufe +IV mit geringer Ausbeute stellt die Oxidation mit NaOCl in DMF dar, siehe J. Org. Chem. 1984, 2282-2284, Inorg. Chem. 2010, 49, 10680-10686.

### Rhodium katalysierte Hydroformylierungen im Vergleich zu Diphenylselenid IIIa und Diphenylselenoxid IIa

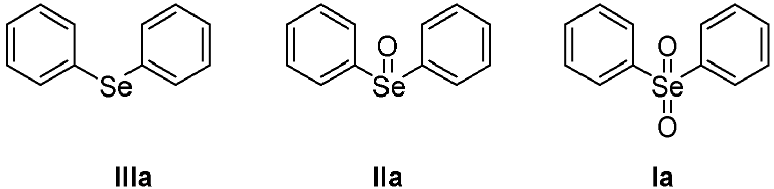

| **Eintrag** | **Ligand** | **Rh [ppm]** | **Olefin/LM** | **p [bar]** | **T [°C]** | **t [h]** | **A [%]** | **S [%]** |
|---|---|---|---|---|---|---|---|---|
| 1 | **IIIa** | 100 | *n*-Octen/ Toluol | 50 | 120 | 4 | 94.3 | 28.5 |
| 2 | **IIa** | 100 | *n*-Octen/ Toluol | 50 | 120 | 4 | 89.5 | 28.1 |
| 3 | **Ia*** | 100 | *n*-Octen/ Toluol | 50 | 120 | 4 | 97.7 | 28.5 |

| | | | | | | | | |
|---|---|---|---|---|---|---|---|---|
| **Erläuterungen zu Tabelle 1:** p = Druck, T = Temperatur, t = Zeit, A = Ausbeute; S = n-Regioselektivität. * = erfindungsgemäße Beispiele | | | | | | | | |

Bei Verwendung von Diphenylselenid **IIIa** konnte eine Ausbeute von 94.3% sowie eine n-Regioselektivität von 28.5% im Katalyse-Experiment erzielt werden.

Bei Verwendung der Organoselen(II)-Verbindung **IIa** konnte eine Ausbeute von 89.5% in der Hydroformylierung mit n-Octenen verzeichnet werden. Dies zeigte im Vergleich zu Verbindung **IIIa** eine etwas verringerte Ausbeute (Vergleich: 94.3%), die n-Regioselektivität wurde annähernd beibehalten.

Die Organoselen(IV)-Verbindung **Ia** zeigte in der Rhodium-katalysierten Hydroformylierung eine verbesserte Ausbeute von 97.7% bei konstanter Ausbildung der n-Selektivität. Die gestellte Aufgabe konnte somit erfüllt werden und diese Komplexe eignen sich in der Verwendung als Katalysatoren von Hydroformylierungsreaktionen.

## Patentansprüche

1. Komplex umfassend
- mindestens ein Diphenylselenon der allgemeinen Struktur **I** 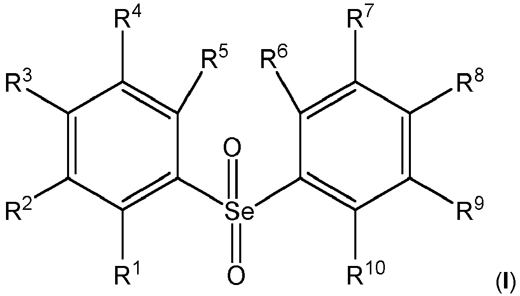 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl,
-CO-(C₆-C₂₀)-Aryl, -COOH, -SO₃H -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- mindestens ein Metallatom ausgewählt aus Rh, Ru, Co, Ir.

2. Komplex nach Anspruch 1,
umfassend ein Diphenylselnon der allgemeinen Struktur **Ia:** 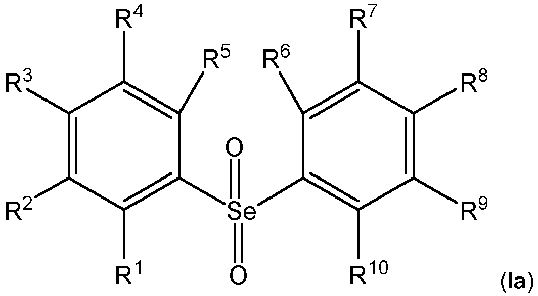
(i) worin R¹, R³, R⁵, R⁶, R⁸ und R¹⁰ jeweils -H und R², R⁴, R⁷ und R⁹ jeweils unabhängig ausgewählt sein können aus -(C₁-C₁₂)-Alkyl, mit Alkyl linear, verzweigt oder cyclisch, oder
(ii) worin R¹, R³, R⁵, R⁶, R⁸ und R¹⁰ jeweils ausgewählt sein können aus -(C₁-C₁₂)-Alkyl, mit Alkyl linear, verzweigt oder cyclisch, und R², R⁴, R⁷ und R⁹ jeweils -H, oder
(iii) worin R³ und R⁸ jeweils ausgewählt sein können aus -O-(C₁-C₁₂)-Alkyl, -O-(C₆-C₂₀)-Aryl, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, und -Halogen, insbesondere -O-(C₂-C₁₂)-Alkyl, bevorzugt -O-(C₃-C₁₂)-Alkyl, mit Alkyl linear, verzweigt oder cyclisch, und R¹, R², R⁴, R⁵, R⁶, R⁷, R⁹ und R¹⁰ jeweils -H, oder
(iv) worin R¹, R², R³, R⁴, R⁵, R⁶, R⁷, R⁸, R⁹, R¹⁰ jeweils unabhängig voneinander ausgewählt sein können aus -Halogen, insbesondere ist Halogen Fluor oder Chlor,
wobei in (i), (ii) (iii) oder (iv) die Alkyl- und Arylgruppen jeweils unabhängig unsubstituiert oder substituiert sein können, substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

3. Komplex nach Anspruch 1,
umfassend ein Diphenylselnon der allgemeinen Struktur **Ib:**

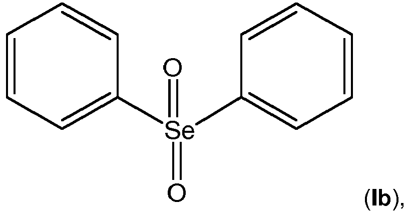

4. Komplex nach Anspruch 1,
umfassend ein Diphenylselnon der allgemeinen Struktur **Ic:** 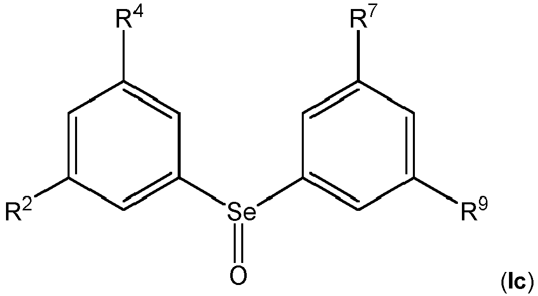 wobei in der Struktur **Ic** R², R⁴, R⁷ und R⁹ jeweils unabhängig ausgewählt sind aus -(C₁-C₁₂)-Alkyl.

5. Komplex nach Anspruch 1,
umfassend ein Diphenylselnon der allgemeinen Struktur **Ic1:**

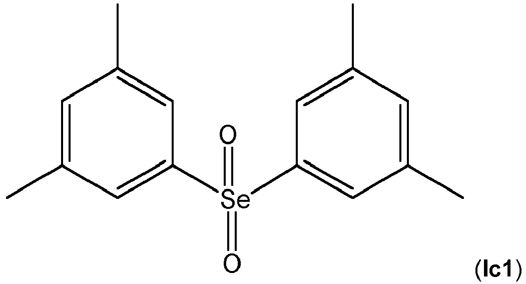

6. Komplex nach Anspruch 1,
umfassend ein Diphenylselnon der allgemeinen Struktur **Id:**

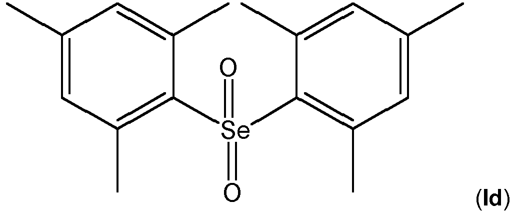

7. Komplex nach Anspruch 1,
umfassend ein Diphenylselnon der allgemeinen Struktur **Ie:** 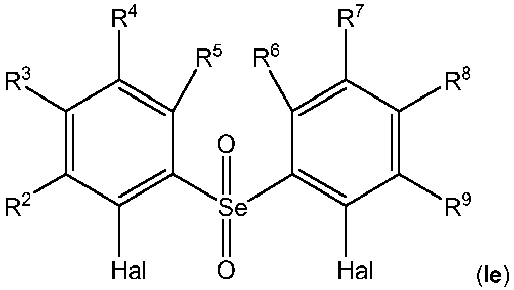 mit R², R³, R⁴, R⁵, R⁶, R⁷, R⁸ und R⁹ jeweils unabhängig ausgewählt aus -H, -(C₁-C₁₂)-Alkyl und -Halogen.

8. Verfahren zur Herstellung des Diphenylselenon der Struktur **I**
nach Anspruch 1, indem ein Diphenylselenid der allgemeinen Struktur **III,** 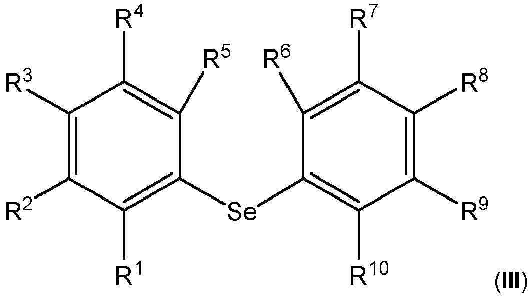 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- in einem ersten aprotischen, polaren, organischen Lösemittel, in Gegenwart eines Alkalisalzes des Hypochlorids oder einem Alkalisalz des Hypobromids zu einem Reaktionsgemisch umgesetzt wird, und
- das Reaktionsgemisch mit einem zweiten organischen Lösemittel unter Ausbildung eines zweiphasigen Gemisches behandelt wird, wobei sich das Diphenylselenon der Struktur **I**, in dem zweiten organischen Lösemittel löst,
- Abtrennen des zweiten organischen Lösemittels enthaltend Diphenylselenon der Struktur **I,**
- Entfernen des zweiten organischen Lösemittels und
- Erhalten des Diphenylselenons der Struktur **I**.

9. Verfahren zur Herstellung des Diphenylselenon der Struktur **I**
nach Anspruch 1, indem ein Diphenylselenoxid der allgemeinen Struktur **II**, 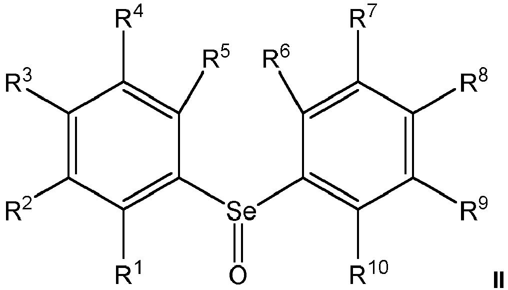 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- in einem aprotischen, organischen Lösemittel in Gegenwart eines mit Alkyl-Gruppen funktionalisierten quartären- Ammoniumhalogenids mit einem Alkalisalz des Hypochlorids oder einem Alkalisalz des Hypobromids, zu einem Reaktionsgemisch umgesetzt wird, und
- das Reaktionsgemisch mit Wasser unter Ausbildung eines zweiphasigen Gemisches behandelt wird, wobei das Diphenylselenon der Struktur **I**, im aprotischen organischen Lösemittel verbleibt,
- Abtrennen des aprotischen, organischen Lösemittels enthaltend Diphenylselenon der Struktur **I,**
- Entfernen des aprotischen, organischen Lösemittels und
- Erhalten des Diphenylselenons der Struktur **I.**

10. Verwendung eines Diphenylselenons der allgemeinen Struktur **I,** 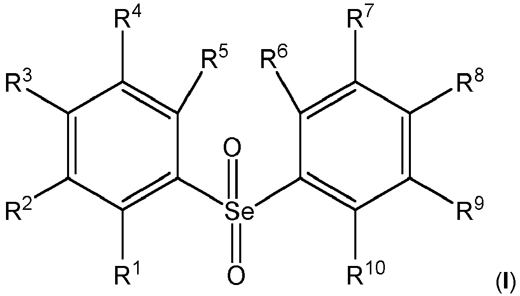 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
a) als Ligand in einem Komplex umfassend mindestens ein Metallatom oder
b) als Zwischenprodukt zur Herstellung von Liganden oder als Zwischenprodukt zur Herstellung von Metall enthaltenden Komplexen,
c) zur Katalyse einer Hydroformylierungsreaktion.

11. Verfahren umfassend die Verfahrensschritte
(i) Vorlegen mindestens eines Olefins,
(ii) Zugabe eines Komplexes nach Anspruch 1 umfassend
- mindestens ein Diphenylselenon, welches eine allgemeine Struktur I aufweist 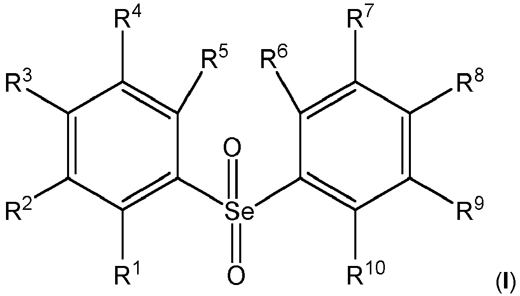 wobei R¹, R², R³, R⁴, R⁵, R⁶, R⁷, R⁸, R⁹, R¹⁰ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=OI-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C₃-C₁₂)-Heterocycloalkyl, -(C₆-C₂₀)-Aryl, Fluor, Chlor, Brom, Cyano, Formyl, Acyl oder Alkoxycarbonyl
- mindestens ein Metallatom ausgewählt aus Rh, Ru, Co, Ir, oder
- Zugabe mindestens eines Diphenylselenons der allgemeinen Struktur I, wie vorstehend beschrieben,
und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.

## Geänderte Patentansprüche

### Geänderte Patentansprüche gemäss Regel 137(2) EPÜ.

1. Verfahren umfassend die Verfahrensschritte
(i) Vorlegen mindestens eines Olefins,
(ii) Zugabe eines Komplexes umfassend
- mindestens ein Diphenylselenon, welches eine allgemeine Struktur I aufweist wobei R¹, R², R³, R⁴, R⁵, R⁶, R⁷, R⁸, R⁹, R¹⁰ jeweils unabhängig voneinander ausgewählt sein können aus: -H, -(C1-C12)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C3-C12)-Heterocycloalkyl, -(C₆-C₂₀)-Aryl, Fluor, Chlor, Brom, Cyano, Formyl, Acyl oder Alkoxycarbonyl
- mindestens ein Metallatom ausgewählt aus Rh, Ru, Co, Ir, oder
- Zugabe mindestens eines Diphenylselenons der allgemeinen Struktur I, wie vorstehend beschrieben,
und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.

## Geänderte Patentansprüche

### Geänderte Patentansprüche gemäss Regel 137(2) EPÜ.

1. Verfahren umfassend die Verfahrensschritte
(i) Vorlegen mindestens eines Olefins,
(ii) Zugabe eines Komplexes umfassend
- mindestens ein Diphenylselenon, welches eine allgemeine Struktur I aufweist 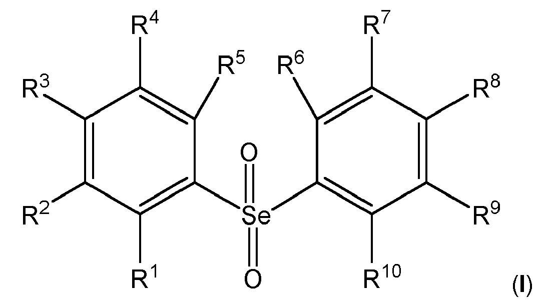 wobei R¹, R², R³, R⁴, R⁵, R⁶, R⁷, R⁸, R⁹, R¹⁰ jeweils unabhängig voneinander ausgewählt sein können aus: -H, -(C1-C12)-Alkyl, -O-(C₁-C₁₂)-Alkyl, -(C₆-C₂₀)-Aryl, -O-(C₆-C₂₀)-Aryl, -Halogen, -O-(C₁-C₁₂)-Alkyl-O-(C₁-C₁₂)-Alkyl, -O-(C₁-C₁₂)-Alkyl-O-(C₆-C₂₀)-Aryl, -O-(C₆-C₂₀)-Aryl-O-(C₁-C₁₂)-Alkyl, -O-(C₆-C₂₀)-Aryl-O-(C₆-C₂₀)-Aryl, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, -NO₂, wobei die Alkyl- und Arylgruppen jeweils unabhängig unsubstituiert oder substituiert sein können; substituierte -(C₁-C₁₂)-Alkylgruppen und substituierte -(C₆-C₂₀)-Arylgruppen können in Abhängigkeit von ihrer Kettenlänge, einen oder mehrere Substituenten aufweisen; die Substituenten können unabhängig voneinander ausgewählt sein unter -(C₃-C₁₂)-Cycloalkyl, -(C3-C12)-Heterocycloalkyl, -(C₆-C₂₀)-Aryl, Fluor, Chlor, Brom, Cyano, Formyl, Acyl oder Alkoxycarbonyl
- mindestens ein Metallatom ausgewählt aus Rh, Ru, Co, Ir, oder
- Zugabe mindestens eines Diphenylselenons der allgemeinen Struktur I, wie vorstehend beschrieben,
und einer Substanz, welche ein Metallatom ausgewählt aus Rh, Ru, Co, Ir aufweist,
(iii) Zuführen von H₂ und CO,
(iv) Erwärmen des Reaktionsgemisches,
wobei das Olefin zu einem Aldehyd umgesetzt wird.
